(19) Europäisches Patentamt
European Patent Office
Office européen des brevets

(11) Publication number: 0 123 735 A1

# EUROPEAN PATENT APPLICATION

(21) Application number: 83200617.5

(22) Date of filing: 29.04.83

(51) Int. Cl.³: C 07 J 13/00, C 07 J 41/00

(43) Date of publication of application: 07.11.84 Bulletin 84/45

(84) Designated Contracting States: NL

(71) Applicant: Gist - Brocades N.V., Wateringseweg 1 P.O. Box 1, NL-2600 MA Delft (NL)

(72) Inventor: van Leusen, Albert Mattheus, De Savornin Lohmanlaan 23, NL-9722 HC Groningen (NL)
Inventor: van Leusen, Adriaan Marinus, Binnensingel 8, NL-9951 GD Winsum (NL)

(74) Representative: Huygens, Arthur Victor, Dr. et al, c/o Gist-Brocades N.V. Patent & Trademarks Department Wateringseweg 1 PO-box 1, NL-2600 MA Delft (NL)

## (54) New process for the preparation of 20-keto-delta-16-steroids and new intermediate compounds formed in this process.

(57) The invention relates to a new process for the introduction into steroids of a 17-side chains containing a 20-keto group under the simultaneously formation of an unsaturated bond between $C_{16}$ and $C_{17}$ by reaction of 17-(isocyanosulfonylmethylene)-steroids with an alkylating agent to 20-isocyano-20-sulfonyl-delta$^{16}$-steroids, followed by hydrolysis of these latter compounds to 20-keto-delta$^{16}$-steroids. The invention also relates to the intermediate compounds which are formed in this process, i.e. 20-isocyano-20-sulfonyl-delta$^{16}$-steroids and to the ultimately formed 20-keto-delta$^{16}$-steroids, as far as these latter compounds are new.

EP 0 123 735 A1

New process for the preparation of 20-keto-delta$^{16}$-steroids and new intermediate compounds formed in this process.

The invention relates to a new process for the preparation of 20-keto-steroids from 17-(isocyano-sulfonylmethylene)-steroids whereby simultaneously an unsaturated bond is introduced between $C_{16}$ and $C_{17}$. Furthermore, the invention relates to new intermediate compounds formed in this process and their preparation.

More particularly, the invention relates to a new process for the introduction into steroids of a 17-side chain containing a 20-keto group under the simultaneous formation of an unsaturated bond between $C_{16}$ and $C_{17}$ by reaction of 17-

(isocyano-sulfonylmethylene)-steroids with an alkylating agent to 20-isocyano-20-sulfonyl-delta$^{16}$-steroids, followed by hydrolysis of these latter compounds to 20-keto-delta$^{16}$-steroids. The invention also relates to the intermediate compounds which are formed in this process, i.e. 20-isocyano-20-sulfonyl-delta$^{16}$-steroids and to the ultimately formed 20-keto-delta$^{16}$-steroids, as far as these latter compounds are new.

The preparation of 17-(isocyano-sulfonylmethylene)--steroids is disclosed in the simultaneously filed patent application No. 0000000, entitled "17-(Isocyano-sulfonyl-methylene)-steroids, 17-(formamido-sulfonylmethylene)-steroids and their preparation", the contents of which have to be regarded as included herein.

The last years much attention has been given to new, cheap raw materials for the synthesis of pharmacologically active steroids. Therefore, the degradation of the abundant soya bean derived sterols sitosterol and campesterol by microbiological methods into 17-oxo-steroids was extensively investigated. As a result thereof 17-oxo-steroids are readily available now against low prices, which makes these compounds ideal starting materials for the synthesis of pharmacologically active steroids.

Due to the fact that almost all pharmacologically active steroids have a 17-side chain, there is a clear need for chemical synthesis whereby 17-side chains in steroids are introduced, starting from 17-oxo-steroids.

In the above mentioned simultaneously filed application a process is given for the introduction of a 17-(isocyano-sulfonylmethylene)-group starting with a 17-oxo-steroid. The present invention now concerns a process whereby the above-mentioned compounds are converted into steroids with a 17-side chain containing a 20-keto group. Pharmacologically active steroids containing a 17-chain in which a 20-keto group is present are for instance progesterons.

It will be appreciated that sometimes 17-side-chains are introduced in steroids to obtain pharmacologically active steroids, or that sometimes further chemical reactions are necessary to obtain the desired side-chains. In the latter case the compounds prepared are intermediates in the preparation of pharmacologically active steroids. Furthermore, it will often be necessary to carry out one or more reactions to obtain the other desired steroid substituents.

The process of the invention is characterized by reacting a 17-(isocyano-sulfonylmethylene)-steroid with an alkylating agent $QR_4$ wherein $R_4$ is an organic radical and Q is a group or atom readily displaced by a nucleophile, followed by hydrolysis.

Furthermore, the invention also relates to a process for the preparation of 20-isocyano-20-sulfonyl-delta$^{16}$-steroids, characterized by reacting a 17-(isocyano-sulfonylmethylene)-steroid with an alkylating agent $QR_4$ wherein $R_4$ is an organic radical and Q is a group or atom readily displaced by a nucleophile.

The above-mentioned 20-isocyano-20-sulfonyl-delta$^{16}$-steroids are intermediates in the preparation of the before-mentioned 20-keto-delta$^{16}$-steroids. These latter compounds can be hydrolized to 20-keto-delta$^{16}$-steroids. Therefore, the invention also relates to a process for the preparation of 20-keto-delta$^{16}$-steroids, characterized by hydrolysis of 20-isocyano-20-sulfonyl-delta$^{16}$-steroids.

Particularly the invention relates to the above-described processes in which the 17-(isocyano-sulfonylmethylene)-steroid has the general formula

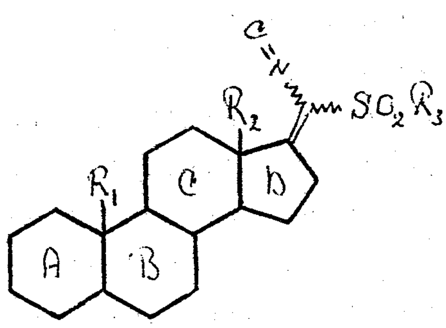

or the 20-isocyano-20-sulfonyl-delta$^{16}$-steroid has the general formula:

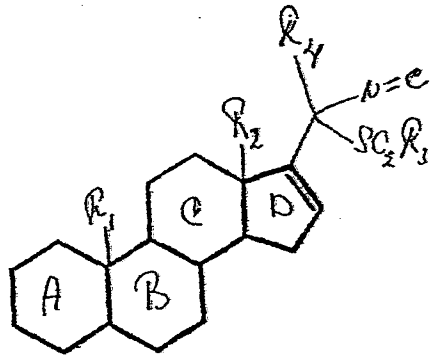

in which $R_1$ represent a hydrogen atom or a methyl group or is absent in the case of a double bond between $C_{10}$ and $C_1$, $C_5$ or $C_9$, $R_2$ represents a hydrogen atom or a methyl group, $R_3$ represents an alkyl or dialkylamino group, or an aryl group substituted or not substituted by one or more halogen atoms, alkyl, alkoxy, nitro or cyano groups, $R_4$ represents an alkyl group optionally containing one or more substituents, and the rings A, B, C and D optionally contain one or more double bonds, are substituted or not substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups, alkylenedioxy, alkylenedithio, or alkyleneoxythio groups.

When $R_3$ represents an alkyl group, suitable alkyl groups are straight or branched alkyl groups having 1-8 carbon atoms, preferably 1-4 atoms.

When $R_3$ represents a dialkylaminogroup, suitable dialkylamino groups are dialkylamino groups wherein the alkyl groups are the same or different and contain 1-8 carbon atoms, preferably 1-4 carbon atoms, or a dialkylamino group wherein the alkyl groups together with the nitrogen atom form a heterocyclic ring which optionally may contain an oxygen atom, the ring containing up to 8 ring atoms. Prefered dialkylamino groups ate dimethylamino, diethyl-amine, pyrolidine and morfoline.

When $R_3$ represents an aryl group, a suitable group is phenyl substituted or not substituted by a halogen atom or an alkyl group, preferably a phenyl or p-methylphenyl group.

When the rings A, B, C and D contain one or more double bonds, these double bonds are preferably present between $C_1$ and $C_2$, $C_3$ and $C_4$, $C_4$ and $C_5$, $C_5$ and $C_6$, $C_6$ and $C_7$, $C_9$ and $C_{10}$, $C_9$ and $C_{11}$ and/or $C_{11}$ and $C_{12}$. More preferably the double bond is present between $C_9$ and $C_{11}$.

When two or more double bonds are present especially the following systems are prefered: $C_3$-$C_4$ and $C_5$-$C_6$, $C_4$-$C_5$ and $C_6$-$C_7$, $C_1$-$C_2$ and $C_4$-$C_5$, $C_1$-$C_2$, $C_3$-$C_4$ and $C_5$-$C_{10}$ and $C_1$-$C_2$, $C_4$-$C_5$ and $C_6$-$C_7$. Preferably there is also a double bond between $C_9$ and $C_{11}$.

When the rings A, B, C and D are substituted by a hydroxy group, suitable groups are 3-, 9-, 11-, 12-, or 14-hydroxy groups.

When the rings A, B, C and D are substituted by an amino group, suitable amino groups are 3-alkylamino groups, preferably containing 1-4 carbon atoms, 3-dialkylamino groups wherein the alkyl groups are the same or different, each alkyl group, preferably containing 1-4 carbon atoms, or amino groups in which the nitrogen atom together with the alkyl group form a heterocyclic ring, preferably containing 1-8 ring atoms, which ring optionally may contain an oxygen atom. Particularly preferred are dimethylamino, diethylamino, pyrolidine and morfoline.

When the rings A, B, C and D are substituted by an oxygen atom this oxygen atom is preferably present at $C_3$, $C_{11}$ or $C_{12}$.

When the rings A, B, C and D are substituted by a halogen atom, suitable halogen atoms are 6-, 9- or 11-fluorine, chlorine or bromine atoms, preferably 6- or 9-fluorine or chlorine atoms.

When the rings A, B, C and D are substituted by an alkyl group, suitable alkyl groups are 1-, 2-, 6-, 7- or 16-methyl groups, preferably 1-, 6- and 16-methyl.

When the rings A, B, C and D are substituted by an alkoxy group, suitable alkoxy groups are 3-, 9-, 11- or 12-alkoxy groups containing 1-4 carbon atoms, preferably 3-, 9-, or 11-methoxy or ethoxy groups.

When the rings A, B, C and D are substituted by an alkoxyalkoxy group, suitable groups are 3- or 11-methoxymethoxy, methoxyethoxy and tetrahydropyranyloxy groups.

When the rings A, B, C and D are disubstituted, suitable substituents are epoxy groups attached to $C_1$ and $C_2$ or $C_9$ and $C_{11}$ or a methylene group attached to $C_1$ and $C_2$ or a 3,3-alkylenedioxy or a 3,3-alkylenedithio group or a 3,3 alkylene oxythiogroup. The alkylene group preferably contains 2 or 3 carbon atom.

More particularly the invention relates to the before mentioned processes for the preparation of the compounds as described before in which $R_1$ and $R_2$ represents methyl, and are substituted by halogen, especially fluorine, or hydroxy at $C_9$ and a hydroxy or keto group at $C_{11}$, or containing functional groups, as a double bond or epoxy group between $C_9$ and $C_{11}$, which can be converted by methods known in the art into the groups mentioned before, and which contain a keto group at $C_3$ and double bonds between $C_1$ and $C_2$ and/or $C_4$ and $C_5$, or containing functional groups which can be converted in the keto group and double bonds as mentioned before.

Suitable alkylating agents $QR_4$ which can be used in the processes of the invention are those alkylating agents in which $R_4$ represents an alkyl group having 1-6 carbon atoms, substituted or not substituted by one or more halogen atoms, an alkoxy or phenylalkoxy group, a cycloalkyl group having 3-8 carbon atoms or a phenyl group, substittuted or not substituted by one or more halogen atoms, alkyl, alkoxy, nitro or cyano groups. Preferably $R_4$ is methyl, halomethyl and benzyloxy.

The reaction of the 17-(isocyano-sulfonylmethylene)-steroid with the alkylating is carried out under basic conditions.

Usually the reaction is carried out with a strong alkaline agent in an organic solvent, preferably in an inert gas atmosphere. Examples of useful strong alkaline agents are alkali metal alcoholates such as alkali metal t-butylates and alkali metal ethanolates, alkali metal hydrides, alkali metal amines, alkali metal alkyls and alkali metal aryls, in which the alkali metal is generally lithium sodium or potassium. Potassium t-butoxide is preferably used. The reaction is preferably carried out at lower temperature, e.g. between -20 and -80°C, preferably between -30 and -60°C, dependant on the solvent used too. The reaction is further preferably carried out in a polar organic solvent such as tetrahydrofuran, dimethylformamide, 1,2-dimethoxyethane, hexamethylfosfortriamide, dioxane, toluene or mixtures thereof. Tetrahydrofuran is preferred. The inert gas atmosphere is preferably a nitrogen or an argon atomosphere.

Futhermore the reaction can also be performed using phase transfer-conditions, i.e. a two phase system of an organic layer and an aqueous layer to which a phase transfer catalyst has been added. Suitable organic solvents for the organic layer are methylenechloride, chloroform, 1,2-dichloroethane, benzene, toluene, chlorobenzene and dichlorobenzene. In general all those organic solvents can be used which are immiscible with water, and in which the relevant compounds are soluble. See for a general survey of phasetransfer reactions E.V. Dhemlov and S.S. Dehmlov, Phase Transfer Catalysis, Weinheim Chemie, 1980.

Suitable aqueous layers are solutions of alkali metal hydroxides in water, for example 5-50% solution of lithium hydroxide, sodium hydroxide or potassium hydroxide. Suitable phase transfer catalysts are quaternary ammonium and fosfonium salts, for instance benzyltrialkyl ammonium halogenide, tetraalkyl ammonium halogenide and alkyltriaryl fosfonium halogenides. The hydrolysis of the 20-isocyano-20-sulfonyl-delta$^{16}$-steroids to the 20-keto-delta$^{16}$-steroids can be carried out in an organic solvent, using an acidic aqueous solution. Suitable organic solvents are for instance diethyl ether, methanol and tetrahydrofuran. Suitable acid are diluted strong acids as hydrogen chloride sulfure acid and fosfor acid. Also acetic acid and formic acid can be used.

It is observed that sometimes not only the 20-isocyano-20-sulfonyl group is hydrolized, but also other groups linked to the steroid skeleton. These groups may have the function of protective groups in the preceding reactions.

The invention also relates to the intermediate 20-isocyano-20-sulfonyl-delta$^{16}$-steroids of the general formula:

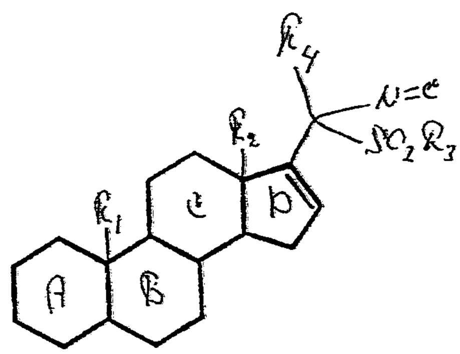

wherein the substituents are as defined above.

Furthermore the invention comprises also the 20-keto-delta$^{16}$-steroids as fas as these cmpounds are new.

The invention is illustrated by the following examples. In the examples THF represents tetrahydrofuran.

The specific rotation of the compounds was measured using light of the sodium D line.

Example 1a

Preparation of 3-methoxy-20-isocyano-20-p-methylphenylsulfonylpregna-3,5,16-triene.

To a solution of 3-methoxy-17-(isocyano-p-methylphenylsulfonylmethylene)-androsta-3,5-diene (954 mg, 2 mmol) in benzene (40 ml), methyl iodide (0.3 ml, 4.8 mmol), benzyltriethylammonium chloride (40 mg) and 50% aqueous NaOH solution (20 ml) were added. The mixture was stirred vigorously for one hour at 80°C. The organic layer was seperated, washed with brine and dried. After evaporation of the solvent the title compound was obtained (910 mg, 93%) m.p. 170-195°C. IR (Nujol) 2180 (N=C), 1665, 1640, 1625, 1610 (C=C + Ar), 1345, 1170 ($SO_2$) $cm^{-1}$. $^1H$ NMR ($CDCl_3$): delta 0.7-2.8 (m), 1.0 (s), 2.0 (2 x s), 2.49 (s), 3.60 (s, 3H), 5.0-5.4 (m, 2H), 6.20 (m, 1H), 7.32, 7.50, 7.82, 7.98 (ABq, 4H).

Example 1b.

Preparation of pregna-4,16-diene-3,20-dione.

The compound prepared in Example 1a (246 mg, 0.5 mmol) was dissolved in diethyl ether (50 ml). After addition of 15% aqueous HCl solution (20 ml) the mixture was stirred vigorously at ambient temperature. The organic layer was separated, washed with brine, washed with saturated $NaHCO_3$-solution and dried ($Na_2SO_4$). After evaporation the title compound was obtained (130 mg, 70%). Melting point (after chromatography and sublimation) 180°C. $(alpha)^{20}$ + 168° (c 1.0, $CHCl_3$) (Litt. A. Wettstein, Helv. Chim. Act. 27, 1803 (1944); m.p. 186-188°C, $(alpha)^{21}$ + 154°(EtOH).

Example 2.

Preparation of 3-methoxypregna-1,3,5(10),16-tetraen-20-one.

3-Methoxy-17-(isocyano-p-methylphenylsulfonylmethylene)-estra-1,3,5(10)-triene (231 mg, 0.5 mmol) was dissolved in DME (3 ml). The solution was cooled till -40°C under a nitrogen atmosphere. After addition of potassium-t-butoxide (100 mg, 0.8 mmol) methyl iodide (142 mg, 0.6 mmol) was added after 10 minutes. The temperature was slowly raised to + 10°C (two hours). The reaction mixture was added to a mixture of diethyl ether (30 ml) and concentrated aqueous hydrogen chloride solution (2 ml), followed by vigorously shaking. After filtration of the organic layer over alumina (act. II - III) and evaporation of the solvent the title compound was obtained (150 mg, 97 %, m.p. 180°C). Crystallization from ethyl acetate afforded the title compound with a melting point of 187°C. $(alpha)^{22}$ + 122°.

Example 3.

Preparation of 3-methoxy-20-isocyano-20-p-methylphenylsulfonyl-21-benzoxypregna-1,3,5(10),16-tetraene.

3-Methoxy-17-(isocyano-p-methylphenylsulfonylmethylene)-estra-1,3,5(10)-triene (230 mg, 0.5 mmol) was dissolved in DME (3 ml). The solution was cooled under a nitrogen atmosphere till -40°C. Potassium-t-butoxide (100 mg) was added followed by chloromethyl benzyl ether (100 mg, 0.64 mmol) after 10 minutes. The temperature was slowly raised (2.5 hours) to + 10°C. The reaction mixture was poored into a saturated solution of $NaHCO_3$, followed by extraction with methylene chloride.

The organic layer was dried and evaporated. The residue was dissolved in methylene chloride and filtrated over alumina (act. II-III). Evaporation of the solvent and crystallization from methanol afforded the title compound (105 mg, 36%), m.p. 153°C (dec.).
IR (Nujol): 2190 (N=C), 1620, 1605, 1585 (Ar + C=C), 1330, 1155 ($SO_2$)$cm^{-1}$. $^1H$ NMR ($CDCl_3$) delta 0.75 (s, 3H), 1.1-3.1 (m), 2.38 (s), 3.7 (s, 3H), 3.99 (s, 0.7H), 4.02 (s, 1.3H), 4.53 (s, 2H), 6.25-6.75 (m, 3H), 6.9-7.4 (m, 8H), 7.64, 7.79 (½ ABq, 2H). Anal. $C_{36}H_{39}NO_4S$ (581.78), calc. C 74.32; H 6.76; N 2.41; S 5.51; found C 73.8; H 6.8; N 2.3; S 5.4.

The same reaction was also performed in the way as described in Example 1a, however at ambient temperature. Yield after crystallization from methanol: 50%.

The title compound can be hydrolized in the way as described in Example 1b to 3-methoxy-21-benzoxypregna-1,3,5(10),16-tetraen-20-one.

## Claims

1. Process for the preparation of 20-keto-delta$^{16}$-steroids, characterized by reacting a 17-(isocyano-sulfonylmethylene)-steroid with an alkylating agent $QR_4$ wherein $R_4$ is an organic radical and Q is a group or atom readily displaced by a nucleophile, followed by hydrolysis.

2. Process for the preparation of 20-isocyano-20-sulfonyl-delta$^{16}$-steroids, characterized by reacting a 17-(isocyano-sulfonylmethylene)-steroid with an alkylating agent $QR_4$ wherein $R_4$ is an organic radical and Q is a group or atom readily displaced by a nucleophile.

3. Process for the preparation of 20-keto-delta$^{16}$-steroids, characterized by hydrolysis of 20-isocyano-20-sulfonyl-delta$^{16}$-steroids.

4. Process according to claims 1-3, characterized in that the 17-(isocyanosulfonylmethylene)-steroid has the general formula:

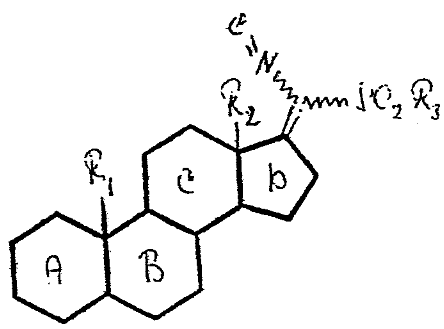

or the 20-isocyano-20-sulfonyl-delta$^{16}$-steroid has the general formula:

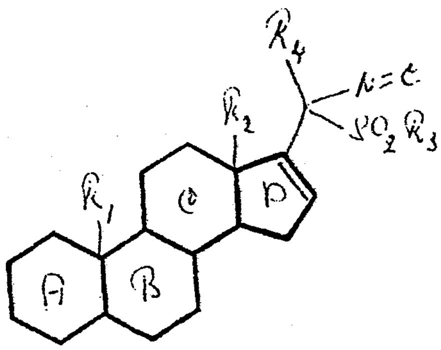

in which $R_1$ represents a hydrogen atom or a methyl group or is absent in the case of a double bond between $C_{10}$ and $C_1$, $C_5$ or $C_9$, $R_2$ represents a hydrogen atom or a methyl group, $R_3$ represents an alkyl or dialkylamino group, or an aryl group substituted or not substituted by one or more halogen atoms, alkyl, alkoxy, nitro or cyano groups, $R_4$ represents an alkyl group optionally containing one or more substituents, and the rings A, B, C and D optionally contain one or more double bonds, are substituted or not substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups, alkylenedioxy, or alkylenedithio or alkyleneoxythio groups.

5. 20-Isocyano-20-sulfonyl-delta$^{16}$-steroids with the general formula:

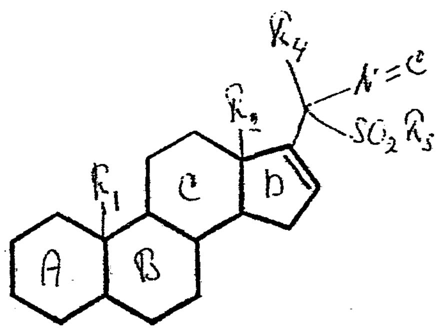

as defined in claim 4.

6. 20-keto-delta$^{16}$-steroids with the general formula:

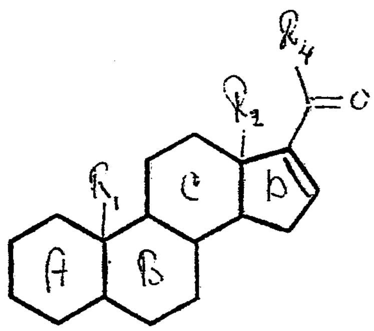

as defined in claim 4.

European Patent Office

# EUROPEAN SEARCH REPORT

0123735

Application number

EP 83 20 0617

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| X | US-A-2 884 418 (FRANZ SONDHEIMER) * Example VI * --- | 6 | C 07 J 13/00<br>C 07 J 41/00 |
| X,D | HELVETICA CHIMICA ACTA, vol. 27, 1944, pages 1803-1814, Basel, CH A. WETTSTEIN: "èber Steroide. Zur Herstellung von 16-Methyl-progesteron und verwandten Verbindungen"* Pages 1805,1810-1813 * --- | 6 | |
| A | GB-A-2 079 756 (ROUSSEL-UCLAF) * Whole document * ----- | 1 | |
| | | | TECHNICAL FIELDS SEARCHED (Int. Cl. 3) |
| | | | C 07 J 13/00<br>C 07 J 41/00 |

The present search report has been drawn up for all claims

| Place of search | Date of completion of the search | Examiner |
|---|---|---|
| THE HAGUE | 15-12-1983 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82